# EUROPEAN PATENT APPLICATION

(11) **EP 0 792 645 A1**
(43) Date of publication of application: **03.09.1997**
(21) Application number: 97301174.5
(22) Date of filing: 21.02.1997
(51) Int. Cl.: A61K 31/565, A61K 31/57, A61K 31/66

(54) **Combination therapy to treat osteoporosis**

(30) Priority: 28.02.1996 US 13367
(71) Applicant: PFIZER INC., New York, N.Y. 10017 (US)
(72) Inventor: MacLean, David B., Providence, Rhode Island 02906 (US); Thompson, David D., Gales Ferry, Connecticut 06335 (US)
(74) Representative: Wood, David John

(57) **Abstract**

A pharmaceutical composition comprising a compound of formula I: 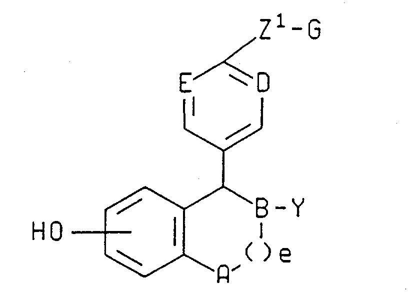 wherein:
A is selected from CH₂ and NR;
B, D and E are independently selected from G, H and N;
e is 0, 1 or 2;
and Y, Z¹ and G are a specified substituent, in combination with a bone resorption inhibiting polyphosphonate or a progestin.
The compositions are useful for treating or preventing osteoporosis.

## Description

### BACKGROUND OF THE INVENTION

Osteoporosis describes a group of diseases which arise from diverse etiologies, but which are characterized by the net loss of bone mass per unit volume. A consequence of this loss of bone mass is the failure of the skeletal frame to provide adequate structural support for the body, resulting in bone fracture. One of the most common types of osteoporosis occurs in women shortly after menopause. Most women lose between 20-60% of the bone mass in the trabecular compartment of the bone within 3-6 years after the cessation of menses. This rapid loss of bone mass is generally associated with an increase of both bone resorption and formation. The resorptive cycle is more dominant, however; and the result is a net loss of bone mass.

Thus, osteoporosis is a common and serious disease among post-menopausal women. An estimated 25 million women in the United States alone are afflicted with this disease. The results of this disease are both personally and economically harmful. Large economic losses are due to its chronic nature and the need for extensive and long term support (hospitalization and nursing home care) from the disease sequelae. The losses are especially great in more elderly patients. Additionally, although osteoporosis is not generally considered a life threatening condition, there is a 20-30% mortality rate related to hip fractures in elderly women. A large percentage of this mortality rate can be directly associated with post-menopausal osteoporosis.

The tissue in the bone most vulnerable to the effects of post-menopausal osteoporosis is the trabecular bone. This tissue is often referred to as spongy or cancellous bone and particularly concentrated near the ends of the bone, near the joints and in the vertebrae of the spine. Trabecular tissue is characterized by small osteoid structures which inter-connect with each other and with the more solid and dense cortical tissue that makes up the outer surface and central shaft of the bone. This criss-cross network of trabeculae gives lateral support to the outer cortical structure and is critical to the bio-mechanical strength of the overall structure. It is primarily the net resorption and loss of the trabeculae which leads to the failure and fracture of bone in post-menopausal osteoporosis. In light of the loss of the trabeculae in post-menopausal women, it is not surprising that the most common fractures are those associated with bones which are highly dependent on trabecular support, e.g., the vertebrae, the neck of the weight bearing bones (femur) and the forearm. Indeed, hip fracture, collies fractures, and vertebral crush fractures are hall-marks of post-menopausal osteoporosis.

A very important concept in the treatment and study of post-menopausal osteoporosis is the concept of fracture threshold. The fracture threshold is the point at which the bone density (therefore, the bone strength) decreases to a value where there is a high probability of bone fracture. This point is not a particular value for all women but rather a relative number for an individual and is dependent on a number of factors such as weight, life-style, or other risks which might contribute to the possibility of bone fracture.

In general, most pre-menopausal women have bone densities above the fracture threshold, and there is a low probability that a fracture will occur. A woman's pre-menopausal bone density and the rate of bone loss after menopause will determine when, or if, she will cross the threshold and be at risk for fracture. For women who present with fractures due to osteoporosis, ideal therapy would be to increase bone density (strength) to a value above the fracture threshold. Alternatively, for women whose bone density is still above the threshold, it would be advantageous to keep them above it.

Today, the only available effective treatment for post-menopausal osteoporosis is hormone replacement therapy, specifically estrogen replacement because post-menopausal women are estrogen deficient. The mechanism of action of estrogen in the treatment of osteoporosis is not well understood; however, it is generally agreed that it inhibits bone resorption. The net effect of the estrogen replacement therapy (ERT) is to keep the woman's bone density at the level at which therapy was initiated, i.e., it maintains bone density. If a woman is above the fracture threshold when (ERT) is initiated, and if ERT is maintained, she will remain above the threshold and be at low risk for fracture. This fact would argue for the placement of women on ERT at or soon after the cessation of menses.

For women whose bone density has already fallen below the fracture threshold, however, ERT will only maintain bone density at the level at which they began therapy. Thus, these women will remain below the threshold and will be at further risk for fracture. ERT is still advisable for these women because it will keep a bad situation from getting worse. It would clearly be advantageous, however, to have a therapy which would boost bone density above the fracture threshold to more normal levels and then maintain it. Currently, there are no effective approved therapies which demonstrate an ability to increase bone density to such a level.

As noted, ERT is now the only effective approved treatment for post-menopausal osteoporosis. In those women who do not have a uterus, estrogen (usually given as a conjugated form of estrone) can be given by itself. In most post-menopausal women who have a uterus, however, unopposed estrogen increases the risk of endometrial cancer. Thus, a progestin is often also administered, either as a combination or in cyclical therapy, to reduce that risk.

"Antiestrogen" is a term that has been rather broadly applied to several different types of compounds that inhibit or modify the action of estrogen. Progestins and androgens have been described as "antiestrongenic..." (Goodman and Gilman, The Pharmacological Basis of Therapeutics, 6th Ed., p 1431.) In addition, certain synthetic compounds, such as tamoxifene, clomiphene, droloxifene and nafoxidine, are called antiestrogens and have been shown both experimentally and clinically to block some of the effects of estrogen. The synthetic "antiestrogens" were principally developed for the treatment of estrogen-dependent breast carcinoma. These compounds are classical mixed agonist/antagonists which demonstrate some estrogenic activity. For example, tamoxifene, the most widely used antiestrogen, has been shown to have estrogenic effects in humans.

The combination of certain 3-benzoyl-benzothiophenes and a progestin has been found to be effective in preventing bone loss. EP 665,015 A2.

European patent EP 0381296 A1 describes the use of a bone cell activating compound in combination with a bone resorption inhibiting polyphosphonate for treatment or prevention of osteoporosis.

Compounds of formula 1 of this invention are useful for treatment of prostate diseases, breast cancer, obesity, cardiovascular disease, hypercholesterolemia and osteoporosis as described in commonly owned United States Patent Application Serial No. 08/369,954 hereby incorporated by reference.

### SUMMARY OF THE INVENTION

The present invention relates to methods for treating or preventing osteoporosis comprising administering to a mammal in need of such treatment an effective amount of a compound of formula I 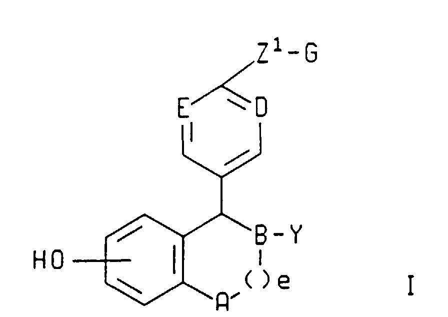 wherein:
A is selected from CH₂ and NR;
B, D and E are independently selected from CH and N;
Y is
   (a) phenyl, optionally substituted with 1-3 substituents independently selected from R⁴;
   (b) naphthyl, optionally substituted with 1-3 substituents independently selected from R⁴;
   (c) C₃-C₈ cycloalkyl, optionally substituted with 1-2 substituents independently selected from R⁴;
   (d) C₃-C₈ cycloalkenyl, optionally substituted with 1-2 substituents independently selected from R⁴;
   (e) a five membered heterocycle containing up to two heteroatoms selected from the group consisting of -O-, -NR²- and -S(O)ₙ-, optionally substituted with 1-3 substituents independently selected from R⁴;
   (f) a six membered heterocycle containing up to two heteroatoms selected from the group consisting of -O-, -NR²- and -S(O)ₙ- optionally substituted with 1-3 substituents independently selected from R⁴; or
   (g) a bicyclic ring system consisting of a five or six membered heterocyclic ring fused to a phenyl ring, said heterocyclic ring containing up to two heteroatoms selected from the group consisting of -O-, -NR²- and -S(O)ₙ-, optionally substituted with 1-3 substituents independently selected from R⁴;
Z¹ is
   (a) -(CH₂)ₚ W(CH₂)_{q}-;
   (b) -O(CH₂)ₚ CR⁵R⁶-;
   (c) -O(CH₂)ₚW(CH₂)_{q};
   (d) -OCHR²CHR³-; or
   (e) -SCHR²CHR³-;
G is
   (a) -NR⁷R⁸;
   (b) 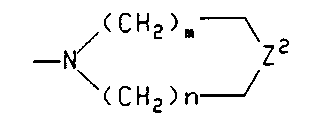 wherein n is 0, 1 or 2; m is 1, 2 or 3; Z² is -NH-, -O-, -S-, or -CH₂-; optionally fused on adjacent carbon atoms with one or two phenyl rings and, optionally independently substituted on carbon with one to three substituents and, optionally, independently on nitrogen with a chemically suitable substituent selected from R⁴; or
   (c) a bicyclic amine containing five to twelve carbon atoms, either bridged or fused and optionally substituted with 1-3 substituents independently selected from R⁴; or
Z¹ and G in combination may be 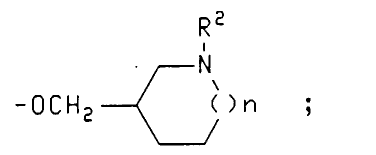
W is
   (a) -CH₂-;
   (b) -CH=CH-;
   (c) -O-;
   (d) -NR²-;
   (e) -S(O)ₙ-;
   (f) 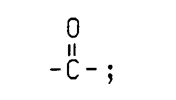
   (g) -CR²(OH)-;
   (h) -CONR²-;
   (i) -NR²CO-;
   (j) 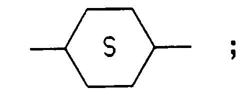 or
   (k) -C ≡ C-;
R is hydrogen or C₁-C₆ alkyl;
R² and R³ are independently
   (a) hydrogen; or
   (b) C₁-C₄ alkyl;
R⁴ is
   (a) hydrogen;
   (b) halogen;
   (c) C₁-C₆ alkyl;
   (d) C₁-C₄ alkoxy;
   (e) C₁-C₄ acyloxy;
   (f) C₁-C₄ alkylthio;
   (g) C₁-C₄ alkylsulfinyl;
   (h) C₁-C₄ alkylsulfonyl;
   (i) hydroxy (C₁-C₄)alkyl;
   (j) aryl (C₁-C₄)alkyl;
   (k) -CO₂H;
   (I) -CN;
   (m) -CONHOR;
   (n) -SO₂NHR;
   (o) -NH₂;
   (p) C₁-C₄ alkylamino;
   (q) C₁-C₄ dialkylamino;
   (r) -NHSO₂R;
   (s) -NO₂;
   (t) -aryl; or
   (u) -OH.
R⁵ and R⁶ are independently C₁-C₈ alkyl or together form a C₃-C₁₀ carbocyclic ring;
R⁷ and R⁸ are independently
   (a) phenyl;
   (b) a C₃-C₁₀ carbocyclic ring, saturated or unsaturated;
   (c) a C₃-C₁₀ heterocyclic ring containing up to two heteroatoms, selected from -O-, -N- and -S-;
   (d) H;
   (e) C₁-C₆ alkyl; or
   (f) form a 3 to 8 membered nitrogen containing ring with R⁵ or R⁶;
R⁷ and R⁸ in either linear or ring form may optionally be substituted with up to three substituents independently selected from C₁-C₆ alkyl, halogen, alkoxy, hydroxy and carboxy;
a ring formed by R⁷ and R⁸ may be optionally fused to a phenyl ring;
e is 0, 1 or 2;
m is 1, 2 or 3;
n is 0, 1 or 2;
p is 0, 1, 2 or 3;
q is 0,1, 2 or 3;
and optical and geometric isomers thereof; and nontoxic pharmacologically acceptable acid addition salts, N-oxides, and quaternary ammonium salts thereof; together with a progestin; or a bone resorption inhibiting polyphosphonate.

Preferred compounds of formula 1 of the invention are of the formula: 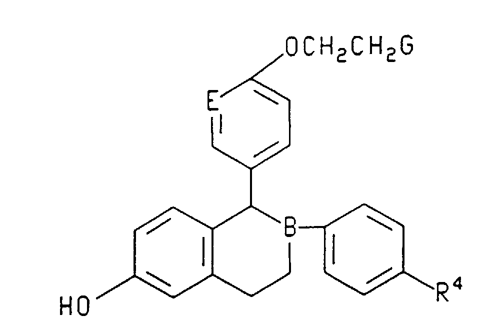 wherein G is 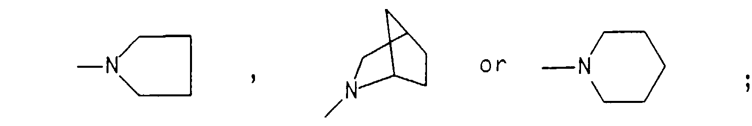 and
R⁴ is H, OH, F, or Cl; and B and E are independently selected from CH and N.

Especially preferred compunds are:
Cis-6-(4-fluoro-phenyl)-5-[4-(2-pipendin-1-yl-ethoxy)-phenyl]-5,6,7,8-tetrahydro-naphthalen-2-ol;
(-)-Cis-6-phenyl-5-[4-(2-pyrrolidin-1-yl-ethoxy)-phenyl]-5,6,7,8-tetrahydro-naphthalen-2-ol;
Cis-6-phenyl-5-[4-(2-pyrrolidin-1-yl-ethoxy)-phenyl]-5,6,7,8-tetrahydro-naphthalen-2-ol;
Cis 1-[6'-pyrrolodinoethoxy-3'-pyridyl]-2-phenyl-6-hydroxy-1,2,3,4-tetrahydrohaphthalene;
1-(4'-Pyrrolidinoethoxyphenyl)-2-(4"-fluorophenyl)-6-hydroxy-1,2,3,4-tetrahydroisoquinoline; and
Cis-6-(4'hydroxyphenyl)-5-[4-(2-piperidin-1-yl-ethoxy)-phenyl]-5,6,7,8-tetrahydro-naphthalen-2-ol

Preferred progestins are: medroxyprogesterone, norethindron and norethylnidene. Alendronate is a preferred polyphosphonate.

### DETAILED DESCRIPTION OF THE INVENTION

The present invention concerns methods for inhibiting bone loss, including treatment and prevention of osteoporosis. The term "inhibit" is defined to include its generally accepted meaning which includes prophylactically treating a subject to prevent the occurrence of one or more of these disease states, holding in check the symptoms of such a disease state, and/or treating such symptoms. Thus, the present methods include both medical therapeutic and/or prophylactic treatment, as appropriate.

The methods of this invention are practiced by administering to an individual in need of treatment an effective amount of a compound formula I above and a progestin or polyphosphate.

The terms C₁-C₃ chloroalkyl and C₁-C₃ fluoroalkyl include methyl, ethyl, propyl and isopropyl substituted to any desired degree with chlorine or fluorine atoms, from one atom to full substitution. The term C₅-C₇ cycloalkyl includes cyclopentyl, cyclohexyl and cycloheptyl.

Halo means chloro, bromo, iodo and fluoro. Aryl (Ar) includes phenyl and naphthyl optionally substituted with one to three substituents independently selected from R⁴ as defined above. DTT means dithiothreitol. DMSO means dimethyl sulfoxide. EDTA means ethylene diamine tetra acetic acid.

Estrogen agonists are herein defined as chemical compounds capable of binding to the estrogen receptor sites in mammalian tissue, and mimicking the actions of estrogen in one or more tissues.

Estrogen antagonists are herein defined as chemical compounds capable of binding to the estrogen receptor sites in mammalian tissue, and blocking the actions of estrogen in one or more tissues.

One of ordinary skill will recognize that certain substituents listed in this invention will be chemically incompatible with one another or with the heteroatoms in the compounds, and will avoid these incompatibilities in selecting compounds of this invention. Likewise certain functional groups may require protecting groups during synthetic procedures which the chemist of ordinary skill will recognize.

The chemist of ordinary skill will recognize that certain compounds of this invention will contain atoms which may be in a particular optical or geometric configuration. All such isomers are included in this invention; exemplary levorotatory isomers in the cis configuration are preferred. Likewise, the chemist will recognize that various pharmaceutically acceptable esters and salts may be prepared from certain compounds of this invention. All of such esters and salts are included in this invention.

The pharmaceutically acceptable salts of the compounds of formula I are salts of non-toxic type commonly used, such as salts with organic acids (e.g., formic, acetic, trifluoroacetic, citric, maleic, tartaric, methanesulfonic, benzenesulfonic or toluenesulfonic acids), inorganic acids (e.g. hydrochloric, hydrobromic, sulfuric or phosphoric acids), and amino acids (e.g., aspartic or glutamic acids). These salts may be prepared by the methods known to chemists of ordinary skill.

Compounds of this invention are readily prepared by the reactions illustrated in the schemes below and described in commonly owned United States Patent Application Serial No. 08/369,954.

Certain compounds of formula I are conveniently prepared from an unsaturated intermediate 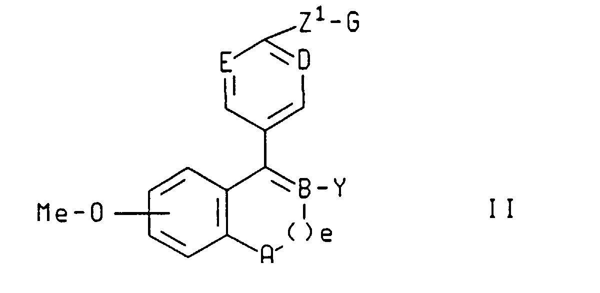 by hydrogenation with a noble metal catalyst in a reaction inert solvent. Pressure and temperatures are not critical and hydrogenation is normally accomplished in a few hours at room temperatures at 20-80 psi hydrogen pressure.

The hydrogenated product is isolated, purified if desired, and the ether group is cleaved with an acidic catalyst in a reaction inert solvent at a temperature between 0°C to 100°C depending on the acidic catalyst used . Hydrogen bromide at elevated temperatures, boron tribromide and aluminum chloride at 0°C to ambient temperature have been found to be effective for this reaction.

The product, Formula I is isolated and purified by standard procedures.

Intermediates of Formula II where A is CH₂, and B, D and E are CH are described in U.S. patent 3,274,213; J. Med. Chem 10, 78 (1967); J. Med. Chem 10, 138 (1967); and J. Med. Chem. 12, 881 (1969), the disclosures of which are herein incorporated by reference. They can also be prepared by procedures described below.

The preparation of the compounds of Formula I where e=1, A=CH₂, Z¹ = OCH₂CH₂, G= cycloalkylamine, B=CH is shown in Scheme 1. Compounds 1-2, where D and E are CH are made by alkylation of 4-bromophenol with the corresponding N-chloroethylamine using potassium carbonate as base in a polar aprotic solvent like dimethylformamide at elevated temperatures. A preferred temperature is 100°C. Compounds 1-2 where D or E or both are N are synthesized using a nucleophilic displacement reaction performed on dibromides (1-1) using hydroxy ethyl cycloalkylamines under phase transfer conditions to afford bromo amines (1-2). Synthesis, 77, 573 (1980). Following halogen metal exchange using n-butyllithium or magnesium metal, bromo amines (1-2) yield the corresponding lithium or magnesium reagents which are allowed to react at low temperature in the presence of cesium chloride preferably (without cesium chloride the reaction also proceeds) with 6-methoxy-1-tetralone to afford either carbinols (1-3) or styrenes (1-4) after acidic workup. Treatment of either carbinols (1-3) or styrenes (1-4) with a brominating agent such as pyridinium bromide perbromide affords bromo styrenes (1-5). Aryl or heteroaryl zinc chlorides or aryl or heteroaryl boronic acids react with bromides (1-5) in the presence of a palladium metal catalyst like tetrakis triphenyl phosphine palladium (0) to yield diaryl styrenes (1-6). [Pure & Applied Chem. 63, 419,(1991) and Bull. Chem. Soc. Jpn. 61, 3008-3010, (1988)] To prepare the preferred compounds the substituted phenyl zinc chlorides or substituted phenylboronic acids are used in this reaction. The aryl zinc chlorides are prepared by quench of the corresponding lithium reagent with anhydrous zinc chloride. The aryl boronic acids, that are not commercially available, are prepared by quenching the corresponding aryl lithium reagent with trialkyl borate, preferably the trimethyl or triisopropyl borate, followed by aqueous acid workup. Acta Chemica Scan. 47, 221-230 (1993). The lithium reagents that are not commercially available are prepared by halogen metal exchange of the corresponding bromide or halide with n-butyl or t-butyllithium. Alternately, the lithium reagent is prepared by heteroatom facilitated lithiations as described in Organic Reactions, Volume 27, Chapter 1. Catalytic hydrogenation of 1-6 in the presence of palladium hydroxide on charcoal, for example, affords the corresponding dihydro methoxy intermediates which were subsequently demethylated using boron tribromide at 0°C in methylene chloride or 48% hydrogen bromide in acetic acid at 80-100°C to afford target structures (1-7). These compounds are racemic and can be resolved into the enantiomers via high pressure liquid chromatography using a column with a chiral stationary phase like the Chiralcel OD columns. Alternately optical resolution can be carried out by recrystallization of the diastereomeric salts formed with optically pure acids like 1,1'-binapthyl-2,2'-diyl hydrogen phosphate.

The cis compounds (1-7) can be isomerized to the trans compounds on treatment with base.

When D and/or E is nitrogen the intermediates (Formula II) and compounds of Formula I may be prepared from the corresponding dihalopyridines or pyrimidines as illustrated in Scheme 1 and as fully described for 6-phenyl-5-[6-(2-pyrrolidin-1-yl-ethoxy) pyridin-3-yl]-5,6,7,8-tetrahydronaphthalen-2-ol in Example 6.

The methyl ether of the compound of Formula I where e=1, A=CH₂, Z¹ = OCH₂CH₂, G= pyrrolidine, D,E, B=CH, Y=Ph can also be conveniently prepared by a first step of hydrogenation of nafoxidine (Upjohn & Co., 700 Portage Road, Kalamazoo, Ml 49001) in a reaction inert solvent in the presence of a nobel metal catalyst. Pressure and temperature are not critical; the reaction is conveniently run in ethanol at room temperature for approximately 20 hours at 50 psi.

The second step is cleavage of the methoxy group which is accomplished conveniently at room temperature with an acidic catalyst such as boron tribromide in a reaction inert solvent or at 80-100°C with hydrogen bromide in acetic acid. The product is then isolated by conventional methods and converted to an acid salt if desired.

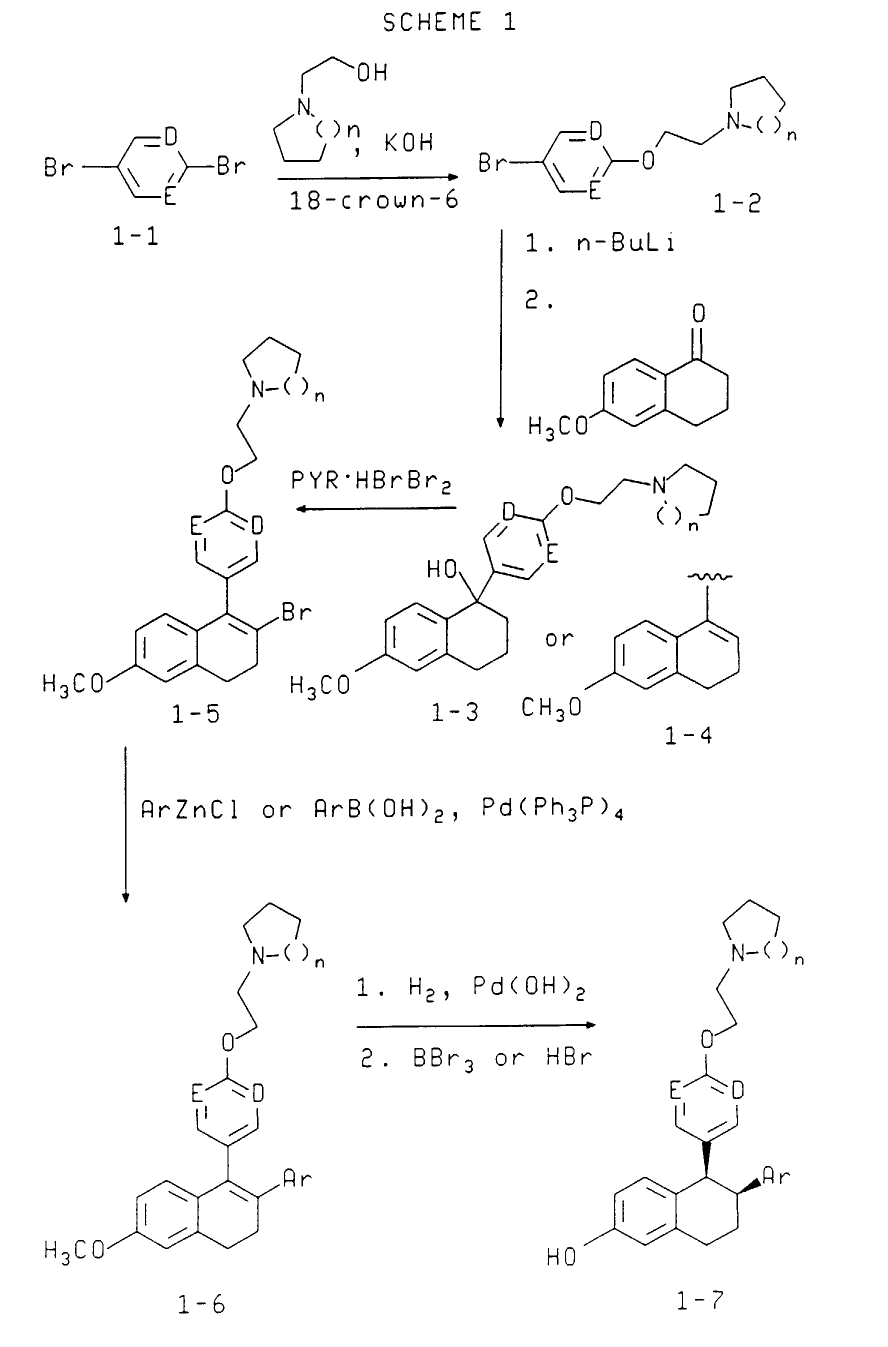

Compounds of formula I wherein B is nitrogen are prepared by the procedures illustrated in Scheme 2 and 3 and Examples 3-5 and 10-12.

The synthesis of compounds of Formula I where B=N is shown in Scheme 2. Aryl acid chlorides (2-1) on treatment with primary amines afford aryl secondary amides (2-2), which are reduced with lithium aluminum hydride in ethereal solvents to yield secondary amines (2-3). Subsequent acylation of (2-3) with aroyl acid chlorides leads to tertiary amides (24), which are cyclized in hot phosphorus oxychloride to yield dihydro isoquinolinium salts (2-5). Reduction with sodium borohydride to alkoxytetrahydro isoquinolines; followed by boron tribromide demethylation in methylene chloride affords the target structures.

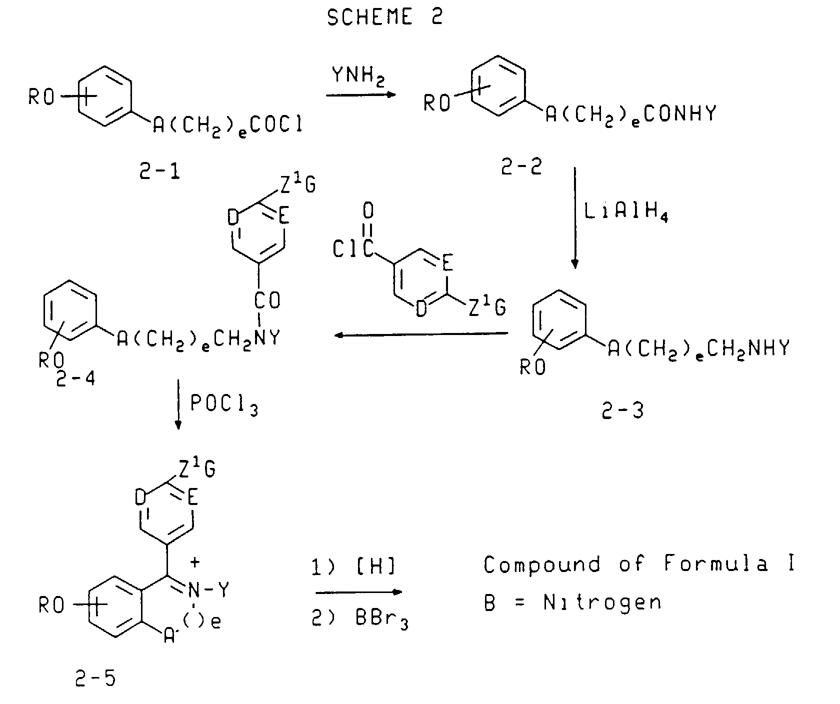

The synthesis of the compounds of Formula I where B=N is also described below in Scheme 3. Secondary amines (3-1) on acylation with benzyloxyaroyl chlorides (3-2) afford tertiary amides (3-3) which on cyclization with hot phosphorous oxychloride yield dihydro isoquinoline salts (3-4). Sodium borohydride reduction of (3-4) followed by debenzylation with aqueous hydrochloric acid affords isoquinolines (3-5), which are alkylated with the appropriately functionalized chlorides and demethylated with boron tribromide to yield the desired target structures.

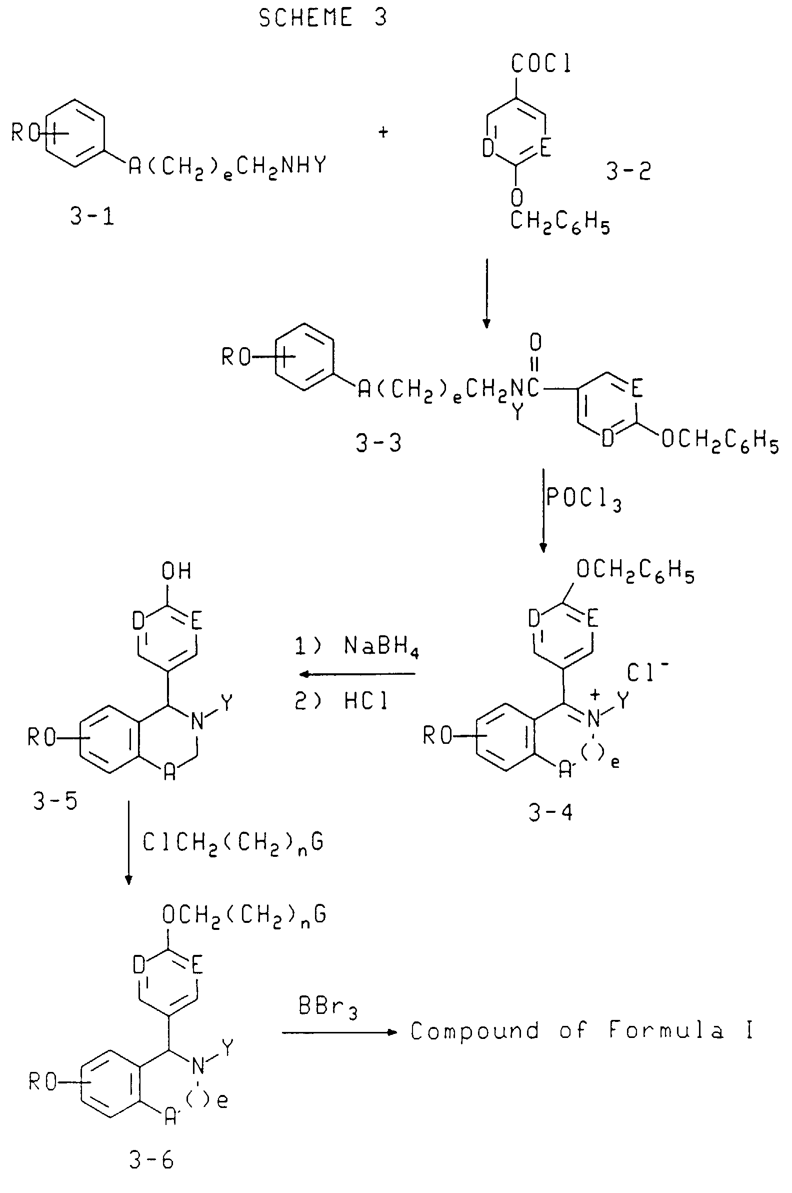

Although the free-base form of formula I compounds can be used in the methods of the present invention, it is preferred to prepare and use a pharmaceutically acceptable salt form. Thus, the compounds used in the methods of this invention form pharmaceutically acceptable acid and base addition salts with a wide variety of inorganic and, preferably, organic acids and include the physiologically acceptable salts which are often used in pharmaceutical chemistry. Such salts are also part of this invention. Typical inorganic acids used to form such salts include hydrochloric, hydrobromic, hydroiodic, nitric, sulfuric, phosphoric, hypophosphoric, and the like. Salts derived from organic acids, such as aliphatic mono and dicarboxylic acids, phenyl substituted alkanoic acids, hydroxyalkanoic and hydroxyalkandioic acids, aromatic acids, aliphatic and aromatic sulfonic acids, may also be used. Such pharmaceutically acceptable salts thus include acetate, phenylacetate, trifluoroacetate, acrylate, ascorbate, benzoate, chlorobenzoate, dinitrobenzoate, hydroxybenzoate, methoxybenzoate, methylbenzoate, o-acetoxybenzoate, naphthalene-2-benzoate, bromide, isobutyrate, phenylbutyrate, β-hydroxybutyrate, butyne-1,4-dioate, hexyne-1 ,4-dioate, caprate, caprylate, chloride, cinnamate, citrate, formate, fumarate, glycollate, heptanoate, hippurate, lactate, malate, maleate, hydroxymaleate, malonate, mandelate, mesylate, nicotinate, isonicotinate, nitrate, oxalate, phthalate, terephthalate, phosphate, monohydrogenphosphate, dihydrogenphosphate, metaphosphate, pyrophosphate, propiolate, propionate, phenylpropionate, salicylate, sebacate, succinate, suberate, sulfate, bisulfate, pyrosulfate, sulfite, bisulfite, sulfonate, benzenesulfonate, p-bromophenylsulfonate, chlorobenzenesulfonate, ethanesulfonate, 2-hydroxyethanesulfonate, methanesulfonate, naphthalene-1-sulfonate, naphthalene-2-sulfonate, p-toluenesulfonate, xylenesulfonate, tartarate, and the like. A preferred salt is the citrate salt.

The pharmaceutically acceptable acid addition salts are typically formed by reacting a compound of formula I with an equimolar or excess amount of acid. The reactants are generally combined in a mutual solvent such as diethyl ether or benzene. The salt normally precipitates out of solution within about one hour to 10 days and can be isolated by filtration or the solvent can be stripped off by conventional means.

The pharmaceutically acceptable salts of formula I compounds generally have enhanced solubility characteristics compared to the compound from which they are derived, and thus are often more amenable to formulation as liquids or emulsions.

Progestins are available from commercial sources and include: Algestone Acetophenide, Altrenogest, Amadinone Acetate, Anagestone Acetate, Chlormadinone Acetate, Cingestol, Clogestone Acetate, Clomegestone Acetate, Delmadinone Acetate, Desogestrel, Dimethisterone, Dydrogesterone, Ethynerone, Ethynodiol Diacetate, Etonogestrel, Flurogestone Acetate, Gestaclone, Gestodene, Gestonorone Caproate, Gestrinone, Haloprogesterone, Hydroxyprogesterone Caproate, Levonorgestrel, Lynestrenol, Medrogestone, Medroxyprogesterone Acetate, Melengestrol Acetate, Methynodiol Diacetate, Norethindrone, Norethindrone Acetate, Norethynodrel, Norgestimate, Norgestomet, Norgestrel, Oxogestone Phenpropionate, Progesterone, Quingestanol Acetate, Quingestrone, and Tigestol.

Preferred progestins are medroxyprogestrone, norethindrone and norethynodrel.

Dosage of progestins is about 0.1 to 10 mg per day; the preferred dose is about 0.25 to 5 mg per day in combination with a compound of formula I.

By "bone resorption inhibiting polyphosphonate" as used herein is meant a polyphosphonate of the type disclosed in U.S. Patent 3,683,080, granted August 8, 1972, the disclosures of which are incorporated herein by reference. Preferred polyphosphonates are geminal diphosphonates (also referred to as bis-phosphonates). The polyphosphonates may be administered in the form of the acid, or of a soluble alkali metal salt or alkaline earth metal salt. Hydrolyzable esters of the polyphosphonates are likewise included. Specific examples include ethane-1-hydroxy 1,1-diphosphonic acid, methane diphosphonic acid, pentane-1-hydroxy-1,1-diphosphonic acid, methane dichloro diphosphonic acid, methane hydroxy diphosphonic acid, ethane-1-amino-1,1-diphosphonic acid, ethane-2-amino-1,1-diphosphonic acid, propane-3-amino-1-hydroxy-1,1-diphosphonic acid, propane-N,N-dimethyl-3-amino-1-hydroxy-1,1-diphosphonic acid, propane-3-3-dimethyl-3-amino-1-hydroxy-1,1-diphosphonic acid, phenyl amino methane diphosphonic acid,N,N-dimethylamino methane diphosphonic acid, N(2-hyroxyethyl) amino methane diphosphonic acid, butane-4-amino-1-hydroxy-1,1 -diphosphonic acid, pentane-5-amino-1-hydroxy-1,1 -diphosphonic acid, hexane-6-amino-1 -hydroxy-1,1-diphosphonic acid and pharmaceutically acceptable esters and salts thereof.

The amount of the polyphosphonate to be used is determined entirely by its potency as a bone resorption inhibiting agent. This potency is determined by means of the thyroparathyroidectomized (TPTX) rat model described herein and expressed as the lowest effective dose (LED) of the compound which is defined as the lowest subcutaneously given dose of polyphosphonate, in mg P per kg body weight, which in the TPTX rat model results in an inhibition of the PTH-induced rise in serum calcium level. Since the amount of polyphosphonate to be administered is dependent on the bone resorption inhibition potency of the compound, the amount to be administered is conveniently expressed as multiples of LED. Extrapolation of the dosages for polyphosphonates from the TPTX rat model to humans is possible based on the observation that oral dosages in humans are proportionally related to the LEDs for polyphosphonates in the TPTX rat model. It is therefore observed that suitable amounts of polyphosphonates for administration in subjects afflicted with or at risk to osteoporosis are from about 0.25 x LED to about 3.3 x LED, while amounts of from about 0.25 x LED to about 2.5 x LED are preferred, and amounts of from 0.50 x LED to 2.0 x LED are most preferred.

Ranges for the daily administration of some polyphosphonates for subjects afflicted with or at risk to osteoporosis are: ethane-1-hydroxy-1,1-diphosphonic acid: from about 0.25 mg P/kg to about 3.3 mg P/kg, with from about 0.25 mg P/kg to about 2.5 mg P/kg preferred; dichloromethane diphosphonic acid: from about 0.12 mg P/kg to about 1.67 mg P/kg, with from about 0.12 mg P/kg to about 1.25 mg P/kg preferred; propane-3-amino-1-hydroxy-1,1 -diphosphonic acid: from about 0.025 mg P/kg to about 0.33 mg P/kg with from about 0.025 mg P/kg to about 0.25 mg P/kg preferred; butane-4-amino-1-hydroxy-1,1-diphosphonic acid: from about 0.0025 mg P/kg to about 0.033 mg P/kg, with from about 0.0025 mg P/kg to about 0.025 mg P/kg preferred; and hexane-6-amino-1-hydroxy-1,1-diphosphonic acid: from about 0.025 mg P/kg to about 0.33 mg P/kg, with from about 0.025 mg P/kg to about 0.25 mg P/kg preferred.

The ranges of daily doses of the above polyphosphonates for use in the present invention are therefore (assuming that the majority of subjects afflicted with or at risk to osteoporosis weigh between about 40 kg to 100 kg): ethane-1-hydroxy-1,1-diphosphonic acid: from about 2.5 mg P to about 330 mg P, with from about 2.5 mg P to about 250 mg P preferred, from about 15 mg P to about 200 mg P more preferred, and from about 15 mg P to about 150 mg P most preferred; dichloromethane diphosphonic acid: from about 1.2 mg P to about 167 mg P, with from about 1.2 mg P to about 125 mg P preferred, from about 7 mg P to about 100 mg P more preferred, and from about 7 mg P to about 75 mg P most preferred: propane-3-amino-1-hydroxy-1,1-diphosphonic acid: from about 0.25 mg P to about 33 mg P, with from about 0.25 mg P to about 25 mg P preferred, from about 1.5 mg P to about 20 mg P more preferred, and from about 1.5 mg P to about 15 mg P most preferred; butane-4-amino-1-hydroxy-1,1 -diphosphonic acid: from about 0.025 mg P to about 3.3 mg P, with from about 0.025 mg P to about 2.5 mg P preferred, from about 0.15 mg P to about 2.0 mg P more preferred, and from about 0.15 mg P to about 1.5 mg P most preferred; and hexane-6-amino-1-hydroxy-1,1-diphosphonic acid: from about 0.25 mg P to about 33 mg P, with from about 0.25 mg P to about 25 mg P preferred, from about 1.5 mg P to about 20 mg P more preferred, and from about 1.5 mg P to about 15 mg P most preferred.

Once prepared, the free base or salt form of formula I compounds together with a bone resorption inhibiting polyphosphonate or progestin can be administered to an individual in need of treatment for the methods herein described. The following nonlimiting test examples illustrate the methods of the present invention.

For the methods of the present invention, compounds of Formula I are administered continuously, or from 1 to 4 times daily together with a bone resorption inhibiting polyphosphonate, or a progestin.

As used herein, the term "effective amount" means an amount of compounds of the methods of the present invention which is capable of inhibiting the symptoms of the pathological conditions herein described. The specific dose of the compounds administered according to this invention will, of course, be determined by the particular circumstances surrounding the case including, for example, the compounds administered, the route of administration, the state of being of the patient, and the severity of the pathological condition being treated. A typical daily dose will contain a nontoxic dosage level of from about 0.25 mg to about 100 mg/day of the compounds of the present invention. Preferred daily doses generally will be from about 1 mg to about 40 mg/day.

The compounds of this invention can be administered by a variety of routes including oral, rectal, transdermal, subucutaneous, intravenous, intramuscular, and intranasal. These compounds preferably are formulated prior to administration, the selection of which will be decided by the attending physician. Typically, a formula I compound, or a pharmaceutically acceptable salt thereof, and a bone resorption inhibiting polyphosphonate are combined with a pharmaceutically acceptable carrier, diluent or excipient to form a pharmaceutical formulation.

The total active ingredients in such formulations comprises from 0.1% to 99.9% by weight of the formulation. By "pharmaceutically acceptable" it is meant the carrier, diluent, excipients, and/or salt must be compatible with the other ingredients of the formulation, and not deleterious to the recipient thereof.

Pharmaceutical formulations containing a compound of formula I and a bone resorption inhibiting polyphosphonate or progestin can be prepared by procedures known in the art using well known and readily available ingredients. For example, the compounds of formula I can be formulated with common excipients, diluents, or carriers, and formed into tablets, capsules, suspensions, powders, and the like. Examples of excipients, diluents, and carriers that are suitable for such formulations include the following fillers and extenders such as starch, sugars, mannitol, and silicic derivatives binding agents such as carboxymethyl cellulose and other cellulose derivatives, alginates, gelatin, and polyvinyl-pyrrolidone; moisturizing agents such as glycerol; disintegrating agents such as calcium carbonate and sodium bicarbonate agents for retarding dissolution such as paraffin resorption accelerators such as quaternary ammonium compounds; surface active agents such as cetyl alcohol, glycerol monostearate; adsorptive carriers such as kaolin and bentonite; and lubricants such as talc, calcium and magnesium stearate, and solid polyethyl glycols.

The compounds also can be formulated as elixirs or solutions for convenient oral administration or as solutions appropriate for parenteral administration, for example, by intramuscular, subcutaneous or intravenous routes.

Additionally, the compounds are well suited to formulation as sustained release dosage forms and the like. The formulations can be so constituted that they release the active ingredient only or preferably in a particular physiological location, possibly over a period of time. The coatings, envelopes, and protective matrices may be made, for example, from polymeric substances or waxes.

Compounds of formula I and polyphosphonates or progestins generally will be administered in a convenient formulation. The following formulation examples only are illustrative and are not intended to limit the scope of the present invention.

In the formulations which follow, "active ingredient" means a compound of formula I, or a salt thereof in combination with a polyphoshonate or a progestin.

### Formulation 1: Gelatin Capsules

Hard gelatin capsules are prepared using the following:

| Ingredient | Quantity (mg/capsule) |
|---|---|
| Active ingredients | 0.25-100 |
| Starch, NF | 0-650 |
| Starch flowable powder | 0-50 |
| Silicone fluid 350 centistokes | 0-15 |

A tablet formulation is prepared using the ingredients below:

### Formulation 2: Tablets

| Ingredient | Quantity (mg/tablet) |
|---|---|
| Active ingredients | 0.25-100 |
| Cellulose, microcrystalline | 200-650 |
| Silicon dioxide, fumed | 10-650 |
| Stearate acid | 5-15 |

The components are blended and compressed to form tablets.

Alternatively, tablets each containing 0.25-100 mg of active ingredients are made up as follows:

### Formulation 3: Tablets

| Ingredient | Quantity (mg/tablet) |
|---|---|
| Active ingredients | 0.25-100 |
| Starch | 45 |
| Cellulose, microcrystalline | 35 |
| Polyvinylpyrrolidone (as 10% solution in water) | 4 |
| Sodium carboxymethyl cellulose | 4.5 |
| Magnesium stearate | 0.5 |
| Talc | 1 |

The active ingredients, starch, and cellulose are passed through a No. 45 mesh U.S. sieve and mixed thoroughly. The solution of polyvinylpyrrolidone is mixed with the resultant powders which are then passed through a No. 14 mesh U.S. sieve. The granules so produced are dried at 50° - 60°C and passed through a No. 18 mesh U.S. sieve. The sodium carboxymethyl starch, magnesium stearate, and talc, previously passed through a No. 60 U.S. sieve, are then added to the granules which, after mixing, are compressed on a tablet machine to yield tablets.

Suspensions each containing 0.25-100 mg of medicaments per 5 ml dose are made as follows:

### Formulation 4: Suspensions

| Ingredient | Quantity (mg/5 ml) |
|---|---|
| Active ingredients | 0.25-100 mg |
| Sodium carboxymethyl cellulose | 50 mg |
| Syrup | 1.25 mg |
| Benzoic acid solution | 0.10 mL |
| Flavor | q.v. |
| Color | q.v. |
| Purified Water to | 5 mL |

The medicaments are passed through a No. 45 mesh U.S. sieve and mixed with the sodium carboxymethyl cellulose and syrup to form smooth paste. The benzoic acid solution, flavor, and color are diluted with some of the water and added, with stirring. Sufficient water is then added to produce the required volume. An aerosol solution is prepared containing the following ingredients:

### Formulation 5: Aerosol

| Ingredient | Quantity (% by weight) |
|---|---|
| Active ingredients | 0.25 |
| Ethanol | 25.75 |
| Propellant 22 (Chlorodifluoromethane) | 70.00 |

The active ingredients are mixed with ethanol and the mixture added to a portion of the propellant 22, cooled to 30°C, and transferred to a filling device. The required amount is then fed to a stainless steel container and diluted with the remaining propellant. The valve units are then fitted to the container.
Suppositories are prepared as follows:

### Formulation 6: Suppositories

| Ingredient | Quantity (mg/suppository) |
|---|---|
| Active ingredients | 250 |
| Saturated fatty acid glycerides | 2,000 |

The active ingredients are passed through a No. 60 mesh U.S. sieve and suspended in the saturated fatty acid glycerides previously melted using the minimal necessary heat. The mixture is then poured into a suppository mold of nominal 2 g capacity and allowed to cool.

An intravenous formulation is prepared as follows:

### Formulation 7: Intravenous Solution

| Ingredient | Quantity |
|---|---|
| Active ingredients | 20 mg |
| Isotonic saline | 1,000 mL |

The solution of the above ingredients is intravenously administered to a patient at a rate of about 1 mL per minute.

### Example 1

In this example, a model of post-menopausal osteoporosis is used in which effects of different treatments upon femur density are determined.

Seventy-five day old female Sprague Dawley rats (weight range of 225 to 275 g) are obtained from Charles River Laboratories (Portage, Ml). They are housed in groups of 3 and have ad libitum access to food (calcium content approximately 1%) and water. Room temperature is maintained at 22.2° ± 1.7° C with a minimum relative humidity of 40%. The photoperiod in the room is 12 hours light and 12 hours dark.

One week after arrival, the rats undergo bilateral ovariectomy under anesthesia (44 mg/kg Ketamine and 5 mg/kg Xylazine (Butler, Indianapolis, IN) administered intramuscularly). Treatment with vehicle or a compound of formula 1 in combination with a progestin or polyphosphonate is initiated either on the day of surgery following recovery from anesthesia or 35 days following the surgery.

Oral dosage is by gavage in 0.5 mL of 1% carboxymethylcellulose (CMC).

Body weight is determined at the time of surgery and weekly during the study, and the dosage is adjusted with changes in body weight. Vehicle-treated ovariectomized (ovex) rats and non-ovariectomized (intact) rats are evaluated in parallel with each experimental group to serve as negative and positive controls.

The rats are treated daily for 35 days (6 rats per treatment group) and sacrificed by decapitation on the 36th day. The 35-day time period is sufficient to allow maximal reduction in bone density, measured as described infra. At the time of sacrifice, the uteri are removed, dissected free of extraneous tissue, and the fluid contents were expelled before determination of wet weight in order to confirm estrogen deficiency associated with complete ovariectomy. Uterine weight is routinely reduced about 75% in response to ovariectomy. The uteri are then placed in 10% neutral buffered formalin to allow for subsequent histological analysis.

The right femurs are excised and scanned at the distal metaphysis 1 mm from the patellar grove with single photon absorptiometry. Results of the densitometer measurements represent a calculation of bone density as a function of the bone mineral content and bone width.

## Claims

1. A pharmaceutical composition comprising a compound of formula 1 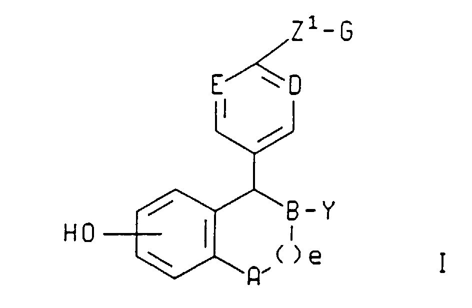 wherein:
A is selected from CH₂ and NR;
B, D and E are independently selected from CH and N;
Y is
(a) phenyl, optionally substituted with 1-3 substituents independently selected from R⁴;
(b) naphthyl, optionally substituted with 1-3 substituents independently selected from R⁴;
(c) C₃-C₈ cycloalkyl, optionally substituted with 1-2 substituents independently selected from R⁴;
(d) C₃-C₈ cycloalkenyl, optionally substituted with 1-2 substituents independently selected from R⁴;
(e) a five membered heterocycle containing up to two heteroatoms selected from the group consisting of -O-, -NR²- and -S(O)ₙ-, optionally substituted with 1-3 substituents independently selected from R⁴;
(f) a six membered heterocycle containing up to two heteroatoms selected from the group consisting of -O-, -NR²- and -S(O)ₙ- optionally substituted with 1-3 substituents independently selected from R⁴; or
(g) a bicyclic ring system consisting of a five or six membered heterocyclic ring fused to a phenyl ring, said heterocyclic ring containing up to two heteroatoms selected from the group consisting of -O-, -NR²-, and -S(O)ₙ-, optionally substituted with 1-3 substituents independently selected from R⁴;
Z¹ is
(a) -(CH₂)ₚ W(CH₂)_{q}-;
(b) -O(CH₂)ₚ CR⁵R⁶-;
(c) -O(CH₂)ₚW(CH₂)_{q};
(d) -OCHR²CHR³-; or
(e) -SCHR²CHR³-;
G is
(a) -NR⁷R⁸;
(b) 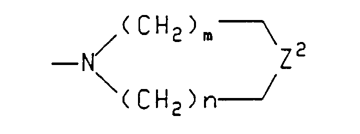 wherein n is 0, 1 or 2; m is 1, 2 or 3; Z² is -NH-, -O-, -S-, or -CH₂-; optionally fused on adjacent carbon atoms with one or two phenyl rings and, optionally independently substituted on carbon with one to three substituents and, optionally, independently on nitrogen with a chemically suitable substituent selected from R⁴; or
(c) a bicyclic amine containing five to twelve carbon atoms, either bridged or fused and optionally substituted with 1-3 substituents independently selected from R⁴;
Z¹ and G in combination may be 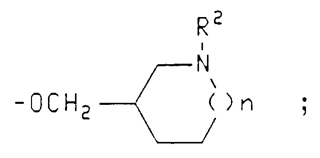
W is
(a) -CH₂-;
(b) -CH =CH-;
(c) -O-;
(d) -NR²-;
(e) -S(O)ₙ-;
(f) 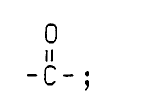
(g) -CR²(OH)-;
(h) -CONR²-;
(i) -NR²CO-;
(j) 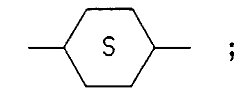 or
(k) -C ≡ C-;
R is hydrogen or C₁-C₆ alkyl;
R² and R³ are independently
(a) hydrogen; or
(b) C₁-C₄ alkyl;
R⁴ is
(a) hydrogen;
(b) halogen;
(c) C₁-C₆ alkyl;
(d) C₁-C₄ alkoxy;
(e) C₁-C₄ acyloxy;
(f) C₁-C₄ alkylthio;
(g) C₁-C₄ alkylsulfinyl;
(h) C₁-C₄ alkylsulfonyl;
(i) hydroxy (C₁-C₄)alkyl;
(j) aryl (C₁-C₄)alkyl;
(k) -CO₂H;
(l) -CN;
(m) -CONHOR;
(n) -SO₂NHR;
(o) -NH₂;
(p) C₁-C₄ alkylamino;
(q) C₁-C₄ dialkylamino;
(r) -NHSO₂R;
(s) -NO₂;
(t) -aryl; or
(u) -OH.
R⁵ and R⁶ are independently C₁-C₈ alkyl or together form a C₃-C₁₀ carbocyclic ring;
R⁷ and R⁸ are independently
(a) phenyl;
(b) a C₃-C₁₀ carbocyclic ring, saturated or unsaturated;
(c) a C₃-C₁₀ heterocyclic ring containing up to two heteroatoms, selected from -O-, -N- and -S-;
(d) H;
(e) C₁-C₆ alkyl; or
(f) form a 3 to 8 membered nitrogen containing ring with R⁵ or R⁶;
R⁷ and R⁸ in either linear or ring form may optionally be substituted with up to three substituents independently selected from C₁-C₆ alkyl, halogen, alkoxy, hydroxy and carboxy;
a ring formed by R⁷ and R⁸ may be optionally fused to a phenyl ring;
e is 0, 1 or 2;
m is 1, 2 or 3;
n is 0, 1 or 2;
p is 0, 1, 2 or 3;
q is 0,1, 2 or 3;
or an optical or geometric isomer thereof; or a non-toxic pharmacologically acceptable acid addition salt, N-oxide, ester, or quaternary ammonium salt thereof; in combination with a bone resorption inhibiting polyphosphonate or a progestin.

2. A composition of claim 1 which the compound of formula 1 is of the formula:

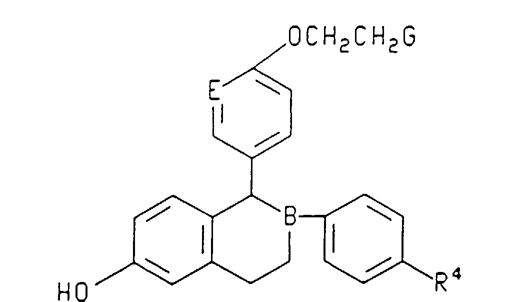

3. A composition of claim 2 wherein G is

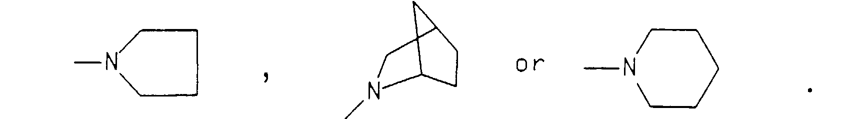

4. A composition of claim 3 wherein R⁴ is H, OH, F or Cl.

5. A composition of claim 4 wherein:
B and E are CH

6. A composition of claim 4 wherein:
B is N; and
E is CH.

7. A composition of claim 4 wherein:
B is CH; and
E is N.

8. A composition of claim 1 in which the compound of formula 1 is
Cis-6-(4-fluoro-phenyl)-5-[4-(2-piperidin-1-yl-ethoxy)-phenyl]-5,6,7,8-tetrahydro-naphthalen-2-ol.

9. A composition of claim 1 in which the compound of formula 1 is
(-)-Cis-6-phenyl-5-[4-(2-pyrrolidin- 1-yl-ethoxy)-phenyl]-5,6,7,8-tetrahydro-naphthalen-2-ol.

10. A composition of claim 1 in which the compound of formula 1 is
Cis-6-phenyl-5-[4-(2-pyrrolidin-1-yl-ethoxy)-phenyl]5,6,7,8-tetrahydro-naphthalen-2-ol.

11. A composition of claim 1 in which the compound of formula I is
Cis 1[6'-pyrrolodinoethoxy-3'-pyridyl]-2-phenyl-6-hydroxy-1,2,3,4-tetrahydronaphthalene.

12. A composition of claim 1 in which the compound of formula I is
1-(4'-Pyrrolidinoethoxyphenyl)-2-(4"-fluorophenyl)-6-hydroxy-1,2,3,4-tetrahydroisoquinoline.

13. A composition of claim 1 in which the compound of formula I is
Cis-6-(4'hydroxyphenyl)-5-[4-(2-piperidin-1-yl-ethoxy)-phenyl]5,6,7,8-tetrahydro-naphthalen-2-ol.

14. A composition of claim 1 in which the compound of formula I is
1-(4'-Pyrrolidinolethoxyphenyl)-2-phenyl-6-hydroxy-1,2,3,4-tetrahydroisoquinoline.

15. A composition of any one of the preceding claims in which the compound of formula I is combined with a bone resorption inhibiting polyphosphonate.

16. A composition of any one of the preceding claims in which the compound of formula I is combined with a progestin.

17. The use of a compound of the formula I as defined in claim 1 or of an optical or geometric isomer thereof; or of a non-toxic pharmacologically acceptable acid addition salt, N-oxide, ester or quaternary ammonium salt thereof; and a bone resorption inhibiting polyphosphonate or a progestin; for the manufacture of a medicament for treating or preventing osteoporosis.

18. The use as claimed in claim 17 wherein the compound of the formula I is as defined in any one of claims 2 to 14.

19. A kit containing a treatment for osteoporosis comprising: -
a therapeutically effective amount of a compound of the formula I or optical or geometric isomer thereof, or non-toxic pharmacologically acceptable acid addition salt, N-oxide, ester or quaternary ammonium salt thereof as defined in any one of claims 1 to 14 and a pharmaceutically acceptable carrier in a first unit dosage form;
a therapeutically effective amount of a bone resorption inhibiting polyphosphonate or a progestin and a pharmaceutically acceptable carrier in a second unit dosage form;
and container means for containing said first and second dosage forms.

20. A kit as claimed in claim 19, composition as claimed in any one of claims 1 to 16, or use as claimed in claim 17 or 18, wherein the polyphosphonate is alendronate.
